(19) Europäisches Patentamt
European Patent Office
Office européen des brevets

(11) Publication number: **0 323 715 B1**

(12) # EUROPEAN PATENT SPECIFICATION

(45) Date of publication of patent specification: **08.09.93**

(51) Int. Cl.$^5$: **A61K 7/06**, A61K 7/11

(21) Application number: **88311559.4**

(22) Date of filing: **07.12.88**

(54) **Hair styling agents and compositions containing the same.**

(30) Priority: **11.12.87 US 131954**

(43) Date of publication of application:
**12.07.89 Bulletin 89/28**

(45) Publication of the grant of the patent:
**08.09.93 Bulletin 93/36**

(84) Designated Contracting States:
**AT BE CH DE FR GB GR IT LI LU NL SE**

(56) References cited:
**EP-A- 320 218**
**EP-A- 0 116 207**
**US-A- 3 927 199**

(73) Proprietor: **THE PROCTER & GAMBLE COMPANY**
**One Procter & Gamble Plaza**
**Cincinnati Ohio 45202(US)**

(72) Inventor: **Bolich, Raymond Edward, Jr.**
**7201 Striker Road**
**Mainville, OH 45039(US)**
Inventor: **Torgerson, Peter Marte**
**4127 U.S. Rt. 35 NW**
**Washington Court House, OH 43160(US)**

(74) Representative: **Brooks, Maxim Courtney et al**
**Procter & Gamble (NTC) Limited Whitley Road Longbenton**
**Newcastle-upon-Tyne NE12 9TS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 323 715 B1

## Description

The present invention relates to rinse-off hair care compositions such as shampoos and conditioners containing water-insoluble styling agents.

The desire to have hair retain a particular shape or configuration is one shared by many people, men and women alike. Approaches taken can either involve permanent or temporary alteration of the hair. The former involves the use of chemical agents to react with the hair in order to achieve the desired effect. This process can be carried out at either room temperature or elevated temperature.

The temporary set given to hair is, as the term indicates, a temporary arrangement which can later be removed by water or by shampooing. The materials used to provide the set have generally been resins or gums. The temporary set compositions have taken the form of gels, lotions, and sprays, and, in more recent years, the form of an aerosol foam (i.e., a styling mousse). The compositions are most often applied to hair dampened with water; then combed or spread throughout the hair by other means; followed by letting the hair dry or blow drying the hair.

The set given will vary depending on the materials used. Temporary set hair styling products typically utilize adhesive polymers which are ethanol or water-soluble rigid polymers having glass transition temperatures well above the temperatures experienced in styling hair. Examples of such high glass transition temperature adhesive polymers are found in US-A-3,743,715 to Viout and Papantoniou, issued July 3, 1973; US-A-4,165,367 to Chakrabarti, issued August 21, 1979; and US-A-4,223,009 to Chakrabarti, issued September 16, 1980. These adhesive polymers are typically applied to the hair in an ethanol or water solvent, and then set to form rigid welds between hair fibers when the solvent evaporates as the hair dries. These hair fiber welds form the basis for the style hold ability of conventional hair styling products. When these welds are broken, they remain broken unless the appropriate polymer solvent is added to redissolve the adhesive and reform the welds when the hair dries.

In addition, many polymers said to be useful in hair styling products are multi-component polymers which combine three, four, and even more monomers into the polymer chains. Frequently, one of the monomer components is vinyl pyrrolidone. Examples of such complex polymer systems are found in US-A-3,222,329 to Grosser et al., issued December 7, 1965; US-A-3,577,517 to Kubot et al., issued May 4, 1971; US-A-4,012,501 to Farber, issued March 15, 1977; and US-A-4,272,511 to Papantoniou and Mondet, issued June 9, 1981.

Other polymers said to be useful for hair styling compositions have been disclosed, such as block polymers. These block polymers have two or more glass transition temperatures. Examples of such block polymer systems are found in US-A-3,907,984 to Calvert et al., issued September 23, 1975; US-A-4,030,512 to Papantoniou et al., issued June 21, 1977; and US-A-4,283,384 to Jacquet et al., issued August 11, 1981. In EP-A-320,218, at least one low glass transition temperature adhesive copolymer is used in combination with a carrier suitable for applying the adhesive copolymer to hair to provide a hair styling composition.

Notwithstanding the great effort already put forth to identify these adhesive polymers for use in temporary set hair styling products, there remains a continuing need to identify new agents which are useful to provide temporary set and other desirable properties to hair. The styling agents of the present invention contain copolymers of two or more monomer components randomly distributed in the copolymer chain along with a volatile diluent. These copolymers have a single glass transition temperature within the temperature range at which hair styling products are typically utilized (i.e., 0 ° C to 150 ° C) and have several properties which make them superior to previously disclosed hair styling polymers for application to hair.

Thus, an object of the present invention is to provide styling agents useful for providing temporary set style hold to hair while remaining pliable on the hair. A further object is to provide styling agents which lengthen the time such temporary set style hold is perceived to be acceptable. A further object is to provide styling agents which provide good temporary set hair style retention while allowing the perception of continued naturalness such as good hair movement and good hair feel. A further object is to provide styling agents which do not make hair feel stiff or sticky. A further object of the present invention is to provide styling agents which give body and/or fullness to hair, and/or which give the ability to provide lift to hair, and/or which increase hair volume. Finally, an object of the present invention is to provide superior rinse-off hair styling compositions comprising the styling agents of the present invention; and to provide an improved method for styling hair by utilizing a hair styling composition of the present invention.

These and other objects of the present invention will become readily apparent from the detailed description which follows.

All percentages and ratios used herein are by weight unless otherwise specified.

According to the present invention there is provided a water-insoluble styling agent comprising:

A. an adhesive copolymer having two or more monomers randomly distributed in a substantially linear copolymer chain having the general structure

$(H_x)_m (L_y)_n$

wherein:

(a) the weight ratio of m:n is within the range of from 10:1 to 1:10;

(b) L is one or more monomer components selected from acrylate esters and methacrylate esters;

(c) H is one or more monomer components selected from acrylate amides and methacrylate amides; and

(d) x and y are integers of 1 or greater, and x + y is an integer of 2 or greater:

and wherein further said adhesive copolymer has a single glass transition temperature within the range of from 0 °C to 150 °C, a number-average molecular weight of from 10,000 to 1,000,000 and a water solubility of less than 0.1%; and

B. a volatile diluent selected from hydrocarbons, esters, ethers, amines, alkyl alcohols and silicon derivatives and mixtures thereof and wherein said diluent has a boiling point of from 99 °C to 260 °C and a water solubility of less than 0.2% at 25 °C;

wherein the weight ratio of adhesive copolymer to volatile diluent is from 1:20 to 5:1.

## Water-Insoluble Styling Active

A. Adhesive Copolymers:

The adhesive copolymer employed in the compositions of the present invention comprises two or more monomers randomly distributed in a copolymer chain (preferably a substantially linear copolymer chain, i.e., having little or no cross-linking or branching of the copolymer chains) such that the copolymer has a single glass transition temperature within the temperature range of from 0 °C to 150 °C, preferably from 0 °C to 80 °C, more preferably from 20 °C to 75 °C, and most preferably from 30 °C to 60 °C.

The monomers to be used for synthesizing the copolymers of the present invention are readily chosen from the groups provided hereinafter based on the hydrophobicity of the monomer and the glass transition temperature of a homopolymer of the monomer. Since the copolymers have two or more component monomers, one or more of the component monomers will be such that it forms a homopolymer having a glass transition temperature above the temperature desired for the copolymer to be synthesized; and one or more of the other component monomers will be such that it forms a homopolymer having a glass transition temperature below the desired glass transition temperature. Combining these monomers randomly in various weight ratios gives copolymers which have single glass transition temperatures between the higher and lower glass transition temperatures for the homopolymers of the monomers utilized.

Thus, simple manipulation of the weight ratios of the monomers during synthesis of the copolymers and appropriate selection of the relative hydrophilicity/hydrophobicity of the monomers utilized, followed by analysis of the resulting copolymers' single glass transition temperatures, permits easy synthesis of copolymers useful in the present invention having the desired combination of single glass transition temperature and solubility.

Monomers which are to be utilized in the present copolymers and which have glass transition temperatures for their homopolymers above and below the desired single glass transition temperature range for the copolymers of the present invention are known, having been disclosed, for example, in US-A-3,907,984 to Calvert, et al., issued September 23, 1975. Monomers having homopolymers with relatively high glass transition temperatures are acrylate amides and methacrylate amides which are preferably unsubstituted or substituted with one or two $C_1$-$C_5$ alkyl groups (i.e., the monomer having the general structure $CH_2=CR(CONR^1R^2)$, wherein R is H or $CH_3$, $R^1$ is H or $C_1$-$C_5$ alkyl, and $R^2$ is H or $C_1$-$C_5$ alkyl). Monomers having homopolymers with relatively low glass transition temperatures are acrylate esters and methacrylate esters which are preferably $C_1$-$C_{15}$ esters of acrylate and methacrylate.

The polymers are selected such that they will dissolve in the water-insoluble diluent and will be soluble at less than 0.1% in water.

The adhesive copolymers employed in compositions of the present invention are random copolymers having the general structure:

$(H_x)_m (L_y)_n$

wherein H is one or more monomer components having homopolymers with relatively high glass transition temperatures as described hereinbefore; L is one or more monomer components having homopolymers with relatively low glass transition temperatures as described hereinbefore; x is the number of different H monomer components present in the copolymer chain, with x being an integer of 1 or greater (preferably x is 1 or 2; most preferably x is 1); y is the number of different L monomer components present in the copolymer chain, with y being an integer of 1 or greater (preferably y is 1 or 2; most preferably y is 2); the sum of x + y is 2 or greater (preferred is x + y being 3); and m:n is the weight ratio of the H monomer components to L monomer components, and is within the range of from 10:1 to 1:10 (preferably from 5:1 to 1:5).

The L monomer components are selected from acrylate esters and methacrylate esters. Preferred acrylate esters and methacrylate esters are the $C_1$-$C_{15}$ esters of acrylate and methacrylate, for example, methylacrylate, ethylacrylate, propylacrylate, butylacrylate, butylmethacrylate, methylmethacrylate, ethylmethacrylate, 2-ethylhexylacrylate, laurylacrylate, and laurylmethacrylate. More preferred L monomer components are isobutylmethacrylate and 2-ethylhexylmethacrylate.

The H monomer components are selected from acrylate amides and methacrylate amides. Preferred are amides of acrylate and methacrylate in which the nitrogen atom is unsubstituted, or substituted with one or two $C_1$-$C_5$ alkyl groups (preferably: methyl, ethyl or propyl), for example, acrylamide, methacrylamide, -N-methylacrylamide, N-methylmethacrylamide, N,N-dimethylmethacrylamide, N-isopropylacrylamide, N-isopropylmethacrylamide and N,N-dimethylacrylamide.

The most preferred adhesive copolymers of the present invention are random copolymers comprising N,N-dimethylacrylamide and two or more acrylate or methacrylate esters having the general structure:

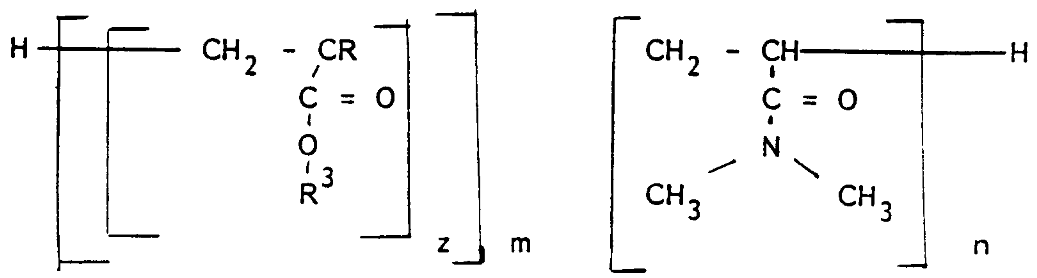

wherein z is the number of different acrylate and methacrylate ester monomer components present in the copolymer chain, with z being an integer of 2 or greater (preferably z = 2); the ratio of m:n is within the range of from 10:1 to 1:1; more preferably from 10:1 to 2:1); R is selected from hydrogen or methyl (preferably R is methyl); and $R^3$ is $C_1$-$C_{15}$ alkyl (preferably $C_1$-$C_{10}$ alkyl). More preferred $R^3$ groups are selected from methyl, ethyl, propyl, butyl, pentyl, and branched alkyl side chains such as isobutyl and 2-ethylhexyl, with most preferred $R^3$ being isobutyl and 2-ethylhexyl.

The term "alkyl", as used herein, means a straight, branched or cyclic carbon-containing chain which is saturated or unsaturated (e.g., one double bond; one triple bond), and which is unsubstituted or substituted with one or more substituent selected from hydroxy, methoxy, ethoxy, propoxy, butoxy, and halogen. Preferred are straight or branched chain, saturated alkyl groups which are unsubstituted.

The adhesive copolymers have a number average molecular weight within the range of from 10,000 to 1,000,000, more preferably within the range of from 10,000 to 150,000, and most preferably from 25,000 to 75,000. It is further preferred that the adhesive copolymers of the present invention have a molecular weight polydispersity (i.e., the ratio of the weight average molecular weight over the number average molecular weight) of 2.5 or less, preferably from 2.5 to 1.0. The adhesive copolymers of the present inventions also preferably have an elastic modulus greater than about $10^6$ Pa. ($10^7$ dynes/$cm^2$), more preferably from about $10^6$ Pa. ($10^7$ dynes/$cm^2$) to about $10^9$ Pa. ($10^{10}$ dynes/$cm^2$), below the copolymer's glass transition temperature.

Analytical methods for analysis of the copolymers of the present invention for their glass transition temperature, number-average molecular weights, molecular weight polydispersity, and elastic modulus are well known in the art. For example, these properties of copolymers and analytical methods are described in more detail in Rosen, Fundamental Principles of Polymeric

Materials (John Wiley & Son, Inc.; New York; 1982).

Synthetic methods for preparing random copolymers having substantially linear chains are well known in the art, for example, US-A-3,222,329 to Grosser, et al., issued December 7, 1965; US-A-3,577,517 to Kubot, et al., issued May 4, 1971; US-A-4,272,511 to Papantoniou and Mondet, issued June 9, 1981; and US-A-4,012,501 to Farber, issued March 15, 1977. Preferably, the copolymers of the present invention are prepared by utilizing free radical polymerization techniques. Typically, such free radical polymerization techniques use either UV wavelength light or chemicals which generate free radicals to initiate the polymerization reaction. Representative procedures for synthesizing low glass transition temperature cationic or nonionic adhesive copolymers of the present invention are provided in the examples hereinafter.

B. Volatile Diluent:

The volatile diluents useful in the present compositions can be hydrocarbons, esters, ethers, amines, alkyl alcohols or silicon derivatives or mixtures thereof and have a boiling point in the range of from 99 °C to 260 °C and have a solubility in water of less than 0.2%. Preferably the volatile diluent is selected from the group of alkyl alcohols and silicon derivatives and mixtures thereof.

The hydrocarbons may be either straight or branched chain and may contain from 10 to 16, preferably from 12 to 16 carbon atoms. Examples of suitable hydrocarbons are decane, dodecane, decene, tridecane and mixtures thereof. Also useful are the terpenes such as orange and lemon terpenes. Useful alkyl alcohols can be saturated or unsaturated and branched or straight chain. Preferred alkyl alcohols include linalool and decyl alcohol.

The volatile silicon derivatives useful in the compositions of the present invention may be either a cyclic or a linear polydialkylsiloxane, linear siloxy compounds or silane. The number of silicon atoms in the cyclic silicones is preferably from 3 to 7, more preferably 3 to 5.

The general formula for such silicones is:

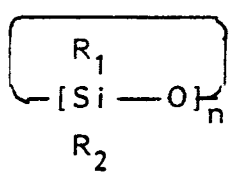

wherein $R_1$ and $R_2$ are independently selected from $C_1$ to $C_8$ alkyl, aryl or alkylaryl and wherein n = 3-7.

The linear polyorgano siloxanes have from 2 to 7 silicon atoms and have the general formula:

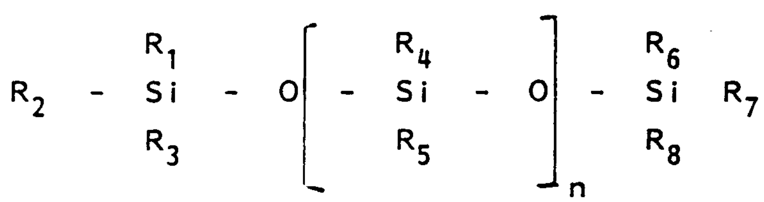

wherein $R_1$, $R_2$, $R_3$, $R_4$, $R_5$, $R_6$, $R_7$, and $R_8$ can independently be saturated or unsaturated $C_1-C_8$ alkyl, aryl, alkyl aryl, hydroxyalkyl, amino alkyl or alkyl siloxy.

Linear siloxy compounds have the general formula:

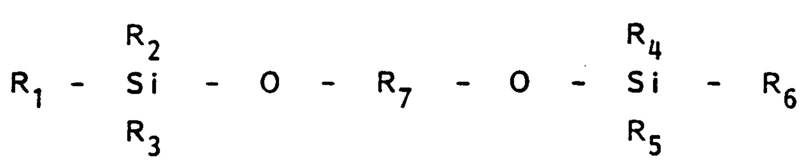

wherein $R_1$, $R_2$, $R_3$, $R_4$, $R_5$ and $R_6$ are independently selected from saturated or unsaturated $C_1$ to $C_7$ alkyl, aryl and alkyl aryl and $R_7$ is $C_1$ to $C_4$ alkylene.

Silane compounds have the general formula:

$$\begin{array}{ccccc} & & R_1 & & \\ R_4 & - & Si & - & R_2 \\ & & R_3 & & \end{array}$$

wherein $R_1$, $R_2$, $R_3$, and $R_4$ can independently be selected from $C_1$-$C_8$ alkyl, aryl, alkyl aryl, hydroxy alkyl and alkylsiloxy.

Silicones of the above type, both cyclic and linear, are offered by Dow Corning Corporation, Dow Corning 344, 345 and 200 fluids, Union Carbide, Silicone 7202 and Silicone 7158, and Stauffer Chemical, SWS-03314.

The linear volatile silicones generally have viscosities of less than about $5 mm^2.s^{-1}$ (5 centistokes) at 25°C while the cyclic materials have viscosities less than about $10 mm^2.s^{-1}$ (10 centistokes). "Volatile" means that the material has a measurable vapor pressure.

A description of volatile silicones is found in Todd and Byers, "Volatile Silicone Fluids for Cosmetics", Cosmetics and Toiletries, Vol. 91, January, 1976, pp. 27-32, and also in Silicon Compounds, pages 253-295, distributed by Petrarch Chemicals.

Optionally, certain water-insoluble non-volatile co-solvents can be added at low levels, i.e., less than about 10%, to replace a similar amount of the volatile diluents. These co-solvents help to dissolve the adhesive copolymer. Preferred co-solvents include liquid alcohols and liquid fatty acids such as isocetyl alcohol and olelyl alcohol.

In order to form the styling agent, the adhesive copolymer and the volatile diluent are combined in a weight ratio of from 1:20 to 5:1, preferably from 1:10 to 1:1 and most preferably from 1:4 to 2:3, the resulting styling agents have an average particle diameter of from 0.5 µm to 100 µm, preferably from 1 µm to 25 µm.

Carriers Suitable for Applying Styling Agents to Hair:

The hair styling compositions of the present invention contain the hair styling agents described above along with a carrier suitable for applying the styling agent to hair. These compositions are of two or more phases; at least one containing the styling agent and another containing the carrier. Other phases can contain, for example, pearlizing agents such as ethylene glycol distearate or $TiO_2$ coated mica which impart aesthetic benefits to the composition. The term "carriers suitable for applying the styling agent to hair", as used herein, means one or more compatible water-based vehicles which are suitable for administration to the hair of a human or lower animal. The term "compatible", as used herein, means that the components of the carrier are capable of being commingled with the adhesive copolymer of the present invention, and with each other, in a manner such that there is no interaction which would substantially reduce the ability of the hair styling compositions to provide temporary set hold to hair under ordinary use situation. These carriers must, of course, be of sufficiently high purity and sufficiently low toxicity to render them suitable for administration to the hair of the human or lower animal to which they are being applied.

Carriers suitable for applying the styling agents such as shampoos and cream rinse conditioners to hair are well known in the art; and their selection can be made without difficulty by a person skilled in the art. For example, carriers which may be selected for use in the hair styling compositions of the present invention are described in more detail in US-A-3,577,517, to Kubot et al, issued May 4, 1971; US-A-3,907,984, to Calvert et al, issued September 23, 1975; US-A-4,012,501, to Farber, issued March 15, 1977; US-A-4,223,009, to Chakrabarti, issued September 16, 1980; and US-A-4,283,384, to Jacquet et al, issued August 11, 1981.

The rinse-off hair styling compositions of the present invention typically comprise water (preferably distilled or deionized), or a water-alcohol mixture (typically in a water:alcohol ratio within the range of from 20:1 to 1:2), as part of the carrier. The carrier is present at a level of from 75% to 99.5%, preferably from 85% to 99%, and most preferably from 90% to 99%.of the total hair styling composition. The hair styling compositions also comprise from 0.5% to 25% of the styling agent of the present invention.

Compositions of this invention also can be formulated in a shampoo form. The shampoos comprise from 1% to 25% of the styling agent; from 5% to 60% of a synthetic surfactant; and the balance water. Suitable surfactants which have been fully described above include ammonium lauryl sulfate, ammonium laureth sulfate, triethylamine lauryl sulfate, triethylamine laureth sulfate, triethanolamine lauryl sulfate,

triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauroyl sulfate, triethanolamine lauroyl sulfate, triethanolamine lauroyl sulfate, monoethanolamine cocoyl sulfate, monoethanolamine lauroyl sulfate, sodium tridecyl benzene sulfonate and sodium dodecyl benzene sulfonate.

These shampoos can contain a variety of nonessential optional components. Such conventional optional ingredients are well known to those skilled in the art, e.g., preservatives, such as benzyl alcohol, ethyl paraben, propyl paraben and imidazolidinyl urea; cationic surfactants, such as cetyl trimethyl ammonium chloride, lauryl trimethyl ammonium chloride, tricetyl methyl ammonium chloride, stearyldimethyl benzyl ammonium chloride, and di(partially hydrogenated tallow) dimethylammonium chloride; thickeners and viscosity modifiers such as a diethanolamide of a long-chain fatty acid (e.g. PEG 3 lauramide), block polymers of ethylene oxide and propylene oxide, sodium chloride, sodium sulfate, polyvinyl alcohol, ethyl alcohol and water-soluble polymers such as xanthan gum, hydroxyethyl cellulose, guar gum and starch; pH adjusting agents, such as citric acid, succinic acid, phosphoric acid, sodium hydroxide, sodium carbonate; perfumes; dyes; and, sequestering agents, such as disodium ethylenediamine tetraacetate. Such agents generally are used individually at a level of from 0.01% to 10%, preferably from 0.5% to 5.0% by weight of the composition.

Where the hair styling compositions are conditioner compositions, preferred optional components include gel vehicle materials. The vehicle preferably comprises two essential components: a lipid vehicle material and generally a cationic surfactant vehicle material. Such gel-type vehicles are generally described in the following documents: Barry, "The Self Bodying Action of the Mixed Emulsifier Sodium Dodecyl Sulfate/Cetyl Alcohol", 28 J. of Colloid and Interface Science 82-91 (1968); Barry, et al., "The Self-Bodying Action of Alkyltrimethylammonium Bromides/Cetostearyl Alcohol Mixed Emulsifiers; Influence of Quaternary Chain Length", 35 J. of Colloid and Interface Science 689-708 (1971); and Barry, et al., "Rheology of Systems Containing Cetomacrogol 1000 - Cetostearyl Alcohol, I. Self Bodying Action", 38 J. of Colloid and Interface Science 616-625 (1972). The lipid vehicle material is preferably used at a level of from 0.1% to 10.0% by weight, while the cationic surfactant vehicle material is preferably used at a level of from 0.05% to 5.0% by weight.

Lipid vehicle materials include naturally or synthetically-derived acids, acid derivatives, alcohols, esters, ethers, ketones, and amides with carbon chains of from 12 to 22, preferably from 16 to 18, carbon atoms in length. Fatty alcohols and fatty esters are preferred; fatty alcohols are particularly preferred.

Lipid vehicle materials among those useful herein are disclosed in Bailey's Industrial Oil and Fat Products, (3d edition, D. Swern, ed. 1979). Fatty alcohols included among those useful herein are disclosed in the following documents: US-A-3,155,591, hilfer, issued November 3, 1964; US-A-4,165,369, Watanabe, et al., issued August 21, 1979; US-A-4,269,824, Villamarin, et al., issued May 26, 1981; GB-A-1,532,585, published November 15, 1978; and Fukushima, et al., "The Effect of Cetostearyl Alcohol in Cosmetic Emulsions", 98 Cosmetics & Toiletries 89-102 (1983). Fatty esters included among those useful herein are disclosed in US-A-3,341,465, Kaufman, et al., issued September 12, 1967.

Preferred esters for use herein include cetyl palmitate and glycerylmonostearate. Cetyl alcohol and stearyl alcohol are preferred alcohols. A particularly preferred lipid vehicle material is comprised of a mixture of cetyl alcohol and stearyl alcohol containing from 55% to 65% (by weight of mixture) of cetyl alcohol.

Other carrier components useful in the hair styling compositions of the present invention are suitable for rendering such compositions more acceptable. These include conventional additives such as opacifiers, colorants, perfumes, UV absorbers, preservatives, medicaments, suds boosters or depressants, penetrants, lustrants, deodorants, and the like. Such carriers are described in more detail in US-A-4,223,009, to Chakrabarti, issued September 16, 1980, and in US-A-4,283,384, to Jacquet et al, issued August 11, 1981.

The hair styling compositions of the present invention typically comprise from 80% to 99.9% of a carrier suitable for applying the adhesive copolymer to hair, preferably from 90% to 99%, and most preferably from 95% to 99%.

The procedure for applying the styling agent to the hair will vary according to the form of the hair styling composition being utilized. For example, hair styling compositions in the form of a shampoo or conditioner lotion are typically applied to the hair when wet, with the hair then being rinsed and dried. An effective amount of the styling agent or composition of the present invention is considered to be an amount sufficient to provide the degree of styling hold desired by the user.

The following examples further describe and demonstrate the preferred embodiments within the scope of the present invention.

## EXAMPLES

The following examples illustrate the present invention. The following adhesive copolymers are used in the examples.

### STYLING POLYMER A

Isobutylmethacrylate/2-ethylhexylmethacrylate/N,N-dimethylacrylamide (60:30:10) copolymer

A 60:30:10 (weight percent) isobutylmethacrylate/2-ethylhexylmethacrylate/N,N-dimethylacrylamide copolymer is prepared as follows. Isobutylmethacrylate, 2-ethylhexylmethacrylate, and N,N-dimethylacrylamide are weighed out (relative weight ratios of 60:30:10) and added to a four neck flask fitted with an argon sparge, mechanical stirrer, thermometer and condenser. Toluene is then added to the flask to get a final monomer concentration of 25.0 weight percent. The reaction flask is placed in a 60 °C water bath and the reaction mixture is sparged with argon for two hours while stirring. After two hours, azobisisobutyronitrile ("AIBN") is added to the reaction flask in an amount sufficient to get a final initiator concentration of 0.25 weight percent. The reaction is then stirred for two hours, after which time the reaction flask is removed from the water bath and the reaction mixture is poured into a polyethylene tray, air dried, and dried in a vacuum oven at 120 °C. Optionally, after two hours of stirring, an additional 0.25% initiator is added and the reaction continued for an additional hour. It is then dried as stated. If it is desired to increase or decrease the polymer molecular weight this is done by altering the level of initiator used, or by adding a chain transfer agent (such as decanethiol) to the reaction mixture. The glass transition temperature of this copolymer is calculated to be approximately 38 °C.

### STYLING POLYMER B

Isobutylmethacrylate/2-ethylhexylmethacrylate/N,N-dimethylacrylamide (30:40:30) copolymer

An isobutylmethacrylate/2-ethylhexylmethacrylate/N,N-dimethylacrylamide copolymer having monomer content in a weight ratio of 30:40:30 is prepared by following essentially the same procedure as described above. The glass transition temperature is calculated to be approximately 44 °C.

### STYLING POLYMER C

Butylmethacrylate/N,N-dimethylacrylamide (80:20) copolymer

A butylmethacrylate/N,N-dimethylacrylamide copolymer having monomer content in a weight ratio of 80:20 is prepared by following essentially the same procedure as described above. The glass transition temperature is calculated to be approximately 30 °C.

EXAMPLE 1

A shampoo composition of the present invention is made by combining the following components.

| Component | Weight % |
|---|---|
| **Styling Agent** | |
| Copolymer C | 02.00 |
| Orange terpenes | 06.00 |
| **Main Mix** | |
| Ammonium lauryl sulfate | 15.00 |
| Jaguar (RTM) HP-60[1] | 01.00 |
| Kathon (RTM) CG[2] | 00.03 |
| Perfume | 00.20 |
| DRO $H_2O$[3] | q.s. |

1) Hydroxypropyl guar gum offered by Hi-tek Polymers, Inc.
2) Preservative offered by Rohm and Haas
3) Double reverse osmosis water

The styling agent (premix) is preblended in a conventional manner known to one skilled in the art. The resulting premix resembles an oil. The premix is then dispersed into the main mix by conventional methods including low shear operations such as a propellar stirrer as well as high shear methods such as colloidal milling.

EXAMPLE II

A Shampoo Composition is made by combining the following components.

| Component | Weight % |
|---|---|
| Styling Agent | |
| Copolymer A | 01.00 |
| 1-decene | 02.00 |
| Main Mix | |
| Ammonium lauryl sulfate | 10.00 |
| Ammonium laureth sulfate | 06.00 |
| Ethylene glycol distearate | 03.00 |
| Cocamide MEA | 01.00 |
| Kathon (RTM) CG | 00.03 |
| DRO $H_2O$ | q.s |

EXAMPLE III

A Shampoo Composition is made by combining the following components.

| Component | Weight % |
|---|---|
| Styling Agent | |
| Copolymer B | 03.00 |
| Cyclomethicone (tetramer) | 09.00 |
| Decyl alcohol | 03.00 |
| Main Mix | |
| Ammonium lauryl sulfate | 11.00 |
| Cocamidopropyl betaine | 04.00 |
| Jaguar (RTM) HP 60 | 01.10 |
| Kathon (RTM) CG | 00.03 |
| DRO $H_2O$ | q.s. |

EXAMPLE IV

A Shampoo Composition is made by combining the following components.

| Component | Weight % |
|---|---|
| Styling Agent | |
| Copolymer A | 00.50 |
| Cyclomethicone (tetramer) | 00.80 |
| Decyl alcohol | 00.20 |
| Premix | |
| Silicone gum[1] | 00.50 |
| Cyclomethicone (tetramer) | 00.60 |
| Main Mix | |
| Ammonium lauryl sulfate | 11.00 |
| Cocamide MEA | 01.50 |
| Xanthan gum | 01.20 |
| Kathon (RTM) CG | 00.03 |
| DRO $H_2O$ | q.s. |

1) G.E. Silicone gum SE-76 offered by General Electric

The styling agent and premix are blended separately and combined with the other ingredients as described above in Example I.

EXAMPLE V

A Styling Rinse Composition is made by combining the following components.

| Component | Weight % |
|---|---|
| Styling Agent | |
| Copolymer A | 02.00 |
| 1-decene | 06.00 |
| Main Mix | |
| Veegum[1] | 01.40 |
| Xanthan gum | 01.40 |
| Cyclomethicone (tetramer) | 00.90 |
| Silicone gum[2] | 00.30 |
| Decyl alcohol | 00.80 |
| Kathon (RTM) CG | 00.03 |
| DRO $H_2O$ | q.s. |

1) Magnesium aluminum silicate offered by R.T. Vanderbilt Co.
2) G.E.S.E. 76

EXAMPLE VI

A Conditioner Composition is made by combining the following components.

| Component | Weight % |
|---|---|
| **Styling Agent** | |
| Copolymer A | 3.00 |
| Phenyl pentamethyl disiloxane | 9.00 |
| **Premix** | |
| Silicone gum[4] | 0.10 |
| Cyclomethicone (pentamer) | 0.50 |
| **Main Mix** | |
| Distearyl dimethyl ammonium chloride | 0.85 |
| Natrosol (RTM) 250M[1] | 0.50 |
| Dow Corning 190[2] | 0.10 |
| Cetyl alcohol | 1.00 |
| Stearyl alcohol | 1.00 |
| Cetareth (RTM) -20 | 0.35 |
| Lexamine (RTM) S-13[3] | 0.50 |
| Perfume | 0.10 |
| Kathon (RTM) CG | 0.03 |
| DRO $H_2O$ | q.s. |

1) Hydroxyethylcellulose offered by Hercules, Inc.
2) A silicone copolyol offered by Dow Corning Corp.
3) a fatty amine offered by Inolex Chemical Division of American Can Company
4) G.E.S.E. 76

EXAMPLE VII

A Conditioner Composition is made by combining the following components.

| Component | Weight % |
|---|---|
| **Styling Agent** | |
| Copolymer A | 0.80 |

| | |
|---|---|
| Hexaethyl disiloxane[1] | 2.00 |
| Isocetyl alcohol | 0.05 |
| Main Mix | |
| Stearalkonium chloride | 1.00 |
| Cetrimonium chloride | 0.50 |
| Cetyl alcohol | 1.20 |
| Stearyl alcohol | 0.50 |
| Ceteth (RTM) -2 | 1.00 |
| Glyceryl monostearate | 0.50 |
| Sodium chloride | 0.05 |
| Kathon (RTM) CG | 0.03 |
| DRO $H_2O$ | q.s. |

1) Supplied by Petrarch Chemical

EXAMPLE VIII

A Conditioner Composition is made by combining the following components.

| Component | Weight % |
|---|---|
| Styling Agent | |
| Copolymer B | 3.00 |
| $D_4$ cyclomethicone | 6.75 |
| Linalool | 2.25 |
| Premix | |
| $D_5$ cyclomethicone | 1.70 |
| Silicone gum[1] | 0.30 |
| Main Mix | |
| Dow Corning 190 silicone surfactant | 0.50 |
| Cetyl alcohol | 0.99 |
| Stearyl alcohol | 0.66 |
| Lexamine (RTM) S-13[2] | 0.50 |
| Ceteareth (RTM) -20 | 0.13 |
| Glycerol monostearate | 0.25 |
| Fragrance | 0.25 |
| Citric acid | 0.09 |
| Kathon (RTM) CG[3] | 0.04 |
| DRO water | q.s. |

1) G.E.S.E. 76
2) Offered by Inolex Chemical Division of American Can Co.
3) Offered by Rohm and Haas Company, Inc.

EXAMPLE IX

A Tonic Composition is made by combining the following components.

| Component | Weight % |
|---|---|
| Styling Agent | |
| Copolymer A | 0.30 |
| Phenyl pentamethyl disiloxane | 1.00 |
| Main Mix | |
| Ethanol, SDA40 | 5.00 |
| Carbopol (RTM) 934[1] | 0.30 |
| Sodium hydroxide | 0.15 |
| Preservative | 0.10 |
| DRO $H_2O$ | q.s. |

[1] Offered by B.F. Goodrich Company.

**Claims**

1. A water-insoluble styling agent comprising:

   A. an adhesive copolymer having two or more monomers randomly distributed in a substantially linear copolymer chain having the general structure

   $(H_x)_m (L_y)_n$

   wherein:

   (a) the weight ratio of m:n is within the range of from 10:1 to 1:10;

   (b) L is one or more monomer components selected from acrylate esters and methacrylate esters;

   (c) H is one or more monomer components selected from acrylate amides and methacrylate amides; and

   (d) x and y are integers of 1 or greater, and x + y is an integer of 2 or greater:

   and wherein further said adhesive copolymer has a single glass transition temperature within the range of from 0°C to 150°C, a number-average molecular weight of from 10,000 to 1,000,000 and a water solubility of less than 0.1%; and

   B. a volatile diluent selected from hydrocarbons, esters, ethers, amines, alkyl alcohols and silicon derivatives and mixtures thereof and wherein said diluent has a boiling point of from 99°C to 260°C and a water solubility of less than 0.2% at 25°C;

   wherein the weight ratio of adhesive copolymer to volatile diluent is from 1:20 to 5:1.

2. A styling agent according to Claim 1 wherein the adhesive copolymer has a glass transition temperature of from 20C to 75°C and a molecular weight of from 25,000 to 75,000.

3. A styling agent according to Claim 2 wherein the volatile diluent is selected from hydrocarbons, silicon derivatives and mixtures thereof.

4. A styling agent according to Claim 3 wherein the weight ratio of adhesive copolymer to volatile diluent is from 1:10 to 1:1 and wherein said styling agent has an average particle diameter of from 1 µm to 25 µm.

5. A styling agent according to Claim 1 wherein the L monomer components of the adhesive copolymer are selected from methylacrylate, ethylacrylate, propylacrylate, butylacrylate, butylmethacrylate, methylmethacrylate, ethylmethacrylate, 2-ethylhexylacrylate, 2-ethylhexylmethacrylate, isobutylacrylate, isobutylmethacrylate, laurylacrylate, laurylmethacrylate, cyclohexylacrylate, and cyclohexylmethacrylate; and the H monomer components are selected from acrylamide, methacrylamide, N-isopropylacrylamide and N,N-dimethylacrylamide; and x + y = 3.

**6.** A styling agent according to claim 5 wherein the weight ratio of m:n is within the range of from 5:1 to 1:5; the L monomer components are selected from isobutylmethacrylate and 2-ethylhexylmethacrylate and mixtures thereof; the H monomer component is N,N-dimethylacrylamide; x is 1; and y is 2.

**7.** A rinse-off hairstyling composition comprising:
A. from 0.5% to 25% by weight of a water-insoluble styling agent according to Claim 1, and
B. from 75% to 99.5% by weight of an aqueous carrier.

**8.** A composition according to Claim 7 wherein said diluent is selected from terpenes, decene, cyclomethicone, dodecane, linalool, pentamethyl disiloxane, hexaethyl disiloxane and mixtures thereof.

**9.** A hair care composition in the form of a shampoo which contains from 1% to 25% by weight of a water-insoluble styling agent according to Claim 1 and from 5% to 60% by weight of a synthetic surfactant or mixture of synthetic surfactants.

**10.** A hair care composition according to Claim 9 wherein the synthetic surfactant is selected from alkyl sulfates, ethoxylated alkyl sulfates and mixtures thereof.

**11.** A hair care composition in the form of a conditioner containing a water-insoluble styling agent according to Claim 1 and which additionally contains from 0.1% to 10.0% by weight of a lipid vehicle material and from 0.05% to 5.0% by weight of a cationic surfactant.

**12.** A hair care composition according to Claim 11 wherein the cationic surfactant is a quaternary ammonium salt.

**13.** A hair care composition according to Claim 12 wherein the lipid vehicle material is selected from cetyl alcohol, stearyl alcohol, cetyl palmitate, glyceryl monostearate and mixtures thereof.

**Patentansprüche**

**1.** Wasserunlösliches Formmittel, umfassend:
A. ein Haftcopolymer aus zwei oder mehreren, in einer im wesentlichen linearen Copolymerkette statistisch verteilten Monomeren, welches Copolymer die allgemeine struktur

$$(H_x)_m\ (L_y)_n$$

besitzt, worin:
(a) das Gewichtsverhältnis von m:n im Bereich von 10:1 bis 1:10 liegt;
(b) L eine oder mehrere, unter Acrylatestern und Methacrylatestern ausgewählte Monomerkomponenten darstellt;
(c) H eine oder mehrere, unter Acrylatamiden und Methacrylatamiden ausgewählte Monomerkomponenten darstellt; und
(d) x und y ganze Zahlen von 1 oder darüber sind, und x + y eine ganze Zahl von 2 oder darüber ist,
und worin das genannte Haftcopolymer ferner eine einzige Glasübergangstemperatur im Bereich von 0 °C bis 150 °C, ein Zahlenmittel-Molekulargewicht von 10.000 bis 1,000.000 und eine Wasserlöslichkeit von weniger als 0,1% aufweist; und
B. ein flüchtiges Verdünnungsmittel, welches unter Kohlenwasserstoffen, Estern, Ethern, Aminen, Alkylalkoholen und Siliconderivaten und Gemischen hievon ausgewählt ist, und worin das genannte Verdünnungsmittel einen Siedepunkt von 99 °C bis 260 °C und eine Wasserlöslichkeit bei 25 °C von weniger als 0,2% aufweist;
worin das Gewichtsverhältnis von Haftcopolymer zum flüchtigen Verdünnungsmittel von 1:20 bis 5:1 beträgt.

**2.** Formmittel nach Anspruch 1, worin das Haftcopolymer eine Glasübergangstemperatur von 20 °C bis 75 °C und ein Molekulargewicht von 25.000 bis 75.000 aufweist.

**3.** Formmittel nach Anspruch 2, worin das flüchtige Verdünnungsmittel unter Kohlenwasserstoffen, Siliconderivaten und Gemischen hievon ausgewählt ist.

**4.** Formmittel nach Anspruch 3, worin das Gewichtsverhältnis von Haftcopolymer zum flüchtigen Verdünnungsmittel von 1:10 bis 1:1 beträgt, und worin das genannte Formmittel einen mittleren Teilchendurchmesser von 1 $\mu$m bis 25 $\mu$m aufweist.

**5.** Formmittel nach Anspruch 1, worin die L-Monomerkomponenten des Haftcopolymers unter Methylacrylat, Ethylacrylat, Propylacrylat, Butylacrylat, Butylmethacrylat, Methylmethacrylat, Ethylmethacrylat, 2-Ethylhexylacrylat, 2-Ethylhexylmethacrylat, Isobutylacrylat, Isobutylmethacrylat, Laurylacrylat, Laurylmethacrylat, Cyclohexylacrylat und Cyclohexylmethacrylat ausgewählt sind, und die H-Monomerkomponenten unter Acrylamid, Methacrylamid, N-Isopropylacrylamid und N,N-Dimethylacrylamid ausgewählt sind, und x + y 3 beträgt.

**6.** Formmittel nach Anspruch 5, worin das Gewichtsverhältnis von m:n im Bereich von 5:1 bis 1:5 beträgt, die L-Monomerkomponenten unter Isobutylmethacrylat und 2-Ethylhexylmethacrylat und Gemischen hievon ausgewählt sind; die H-Monomerkomponente N,N-Dimethylacrylamid ist; x den Wert 1 besitzt und y den Wert 2 hat.

**7.** Abspülbare Haarformzusammensetzung, umfassend:
A. 0,5 Gew.-% bis 25 Gew.-% eines wasserunlöslichen Formmittels nach Anspruch 1 und
B. 75 Gew.-% bis 99,5 Gew.-% eines wäßrigen Trägers.

**8.** Zusammensetzung nach Anspruch 7, worin das genannte Verdünnungsmittel von Terpenen, Decen, Cyclomethicon, Dodecan, Linalool, Pentamethyldisiloxan, Hexaethyldisiloxan und Gemischen hievon ausgewählt ist.

**9.** Haarpflegezusammensetzung in Shampooform, welche 1 Gew.-% bis 25 Gew.-% eines wasserunloslichen Formmittels nach Anspruch 1 und 5 Gew.-% bis 60 Gew.-% eines synthetischen grenzflächenaktiven Mittels oder eines Gemisches aus synthetischen grenzflächenaktiven Mitteln enthält.

**10.** Haarpflegezusammensetzung nach Anspruch 9, worin das synthetische grenzflächenaktive Mittel von Alkylsulfaten, ethoxylierten Alkylsulfaten und Gemischen hievon ausgewählt ist.

**11.** Haarpflegezusammensetzung in der Form eines Konditionierungsmittels, welche ein wasserunlösliches Formmittel nach Anspruch 1 enthält, und zusätzlich 0,1 Gew.-% bis 10,0 Gew.-% eines Lipidträgermaterials und 0,05 Gew.-% bis 5,0 Gew.-% eines kationischen grenzflächenaktiven Mittels beinhaltet.

**12.** Haarpflegezusammensetzung nach Anspruch 11, worin das kationische grenzflächenaktive Mittel ein quaternäres Ammoniumsalz ist.

**13.** Haarpflegezusammensetzung nach Anspruch 12, worin das Lipidträgermaterial aus Cetylalkohol, Stearylalkohol, Cetylpalmitat, Glycerylmonostearat und Gemischen hievon ausgewählt ist.

**Revendications**

**1.** Agent coiffant insoluble dans l'eau, comprenant :
A. un copolymère adhésif possédant au moins deux monomères distribués au hasard dans une chaîne copolymère sensiblement linéaire de structure générale

$(H_x)_m (L_y)_n$

dans laquelle :
(a) le rapport pondéral m:n est dans la gamme de 10:1 à 1:10;

(b) L est un ou plusieurs constituants monomères choisis parmi les esters acrylates et les esters méthacrylates;

(c) H est un ou plusieurs constituants monomères choisis parmi les acrylamides et les méthacrylamides; et

(d) x et y sont des nombres entiers égaux ou supérieurs à 1 et x + y est un nombre entier égal ou supérieur à 2;

et dans lequel, en outre, ledit copolymère adhésif possède une température de transition vitreuse unique dans la gamme de 0 °C à 150 °C, une masse moléculaire moyenne en nombre de 10 000 à 1 000 000 et une solubilité dans l'eau inférieure à 0,1%; et

B. un diluant volatil choisi parmi les hydrocarbures, les esters, les éthers, les amines, les alcools alkyliques et les dérivés du silicium, et leurs mélanges, et dans lequel ledit diluant possède un point d'ébullition de 99 °C à 260 °C et une solubilité dans l'eau inférieure à 0,2% à 25 °C;

dans lequel le rapport pondéral du copolymère adhésif au diluant volatil est de 1:20 à 5:1.

**2.** Agent coiffant selon la revendication 1, dans lequel le copolymère adhésif possède une température de transition vitreuse de 20 °C à 75 °C et une masse moléculaire de 25 000 à 75 000.

**3.** Agent coiffant selon la revendication 2, dans lequel le diluant volatil est choisi parmi les hydrocarbures, les dérivés du silicium et leurs mélanges.

**4.** Agent coiffant selon la revendication 3, dans lequel le rapport pondéral du copolymère adhésif du diluant volatil est de 1:10 à 1:1 et dans lequel ledit agent coiffant a un diamètre moyen de particules de 1 µm à 25 µm.

**5.** Agent coiffant selon la revendication 1, dans lequel les constituants monomères L du copolymère adhésif sont choisis parmi l'acrylate de méthyle, l'acrylate d'éthyle, l'acrylate de propyle, l'acrylate de butyle, le méthacrylate de butyle, le méthacrylate de méthyle, le méthacrylate d'éthyle, l'acrylate de 2-éthylhexyle, le méthacrylate de 2-éthylhexyle, l'acrylate d'isobutyle, le méthacrylate d'isobutyle, l'acrylate de lauryle, le méthacrylate de lauryle, l'acrylate de cyclohexyle et le méthacrylate de cyclohexyle; et les constituants monomères H sont choisis parmi l'acrylamide, le méthacrylamide, le N-isopropylacrylamide et le N,N-diméthylacrylamide; et x + y = 3.

**6.** Agent coiffant selon la revendication 5, dans lequel le rapport pondéral m:n est dans la gamme de 5:1 à 1:5; les constituants monomères L sont choisis parmi le méthacrylate d'isobutyle et le méthacrylate de 2-éthylhexyle et leurs mélanges; le constituant monomère H est le N,N-diméthylacrylamide; x est égal à 1; et y est égal à 2.

**7.** Composition coiffante à rincer, comprenant :

A. de 0,5% à 25% en poids d'un agent coiffant insoluble dans l'eau selon la revendication 1, et

B. de 75% à 99,5% en poids d'un véhicule aqueux.

**8.** Composition selon la revendication 7, dans laquelle ledit diluant est choisi parmi les terpènes, le décène, la cyclométhicone, le dodécane, le linalol, le pentaméthyldisiloxane, l'hexaéthyldisiloxane et leurs mélanges.

**9.** Composition pour le soin des cheveux sous la forme d'un shampooing qui contient de 1% à 25% en poids d'un agent coiffant insoluble dans l'eau selon la revendication 1 et de 5% à 60% en poids d'un tensioactif synthétique ou d'un mélange de tensioactifs synthétiques.

**10.** Composition pour le soin des cheveux selon la revendication 9, dans laquelle le tensioactif synthétique est choisi parmi les alkylsulfates, les alkylsulfates éthoxylés et leurs mélanges.

**11.** Composition pour le soin des cheveux sous forme d'un conditionneur contenant un agent coiffant insoluble dans l'eau selon la revendication 1, et qui contient, en outre, de 0,1% à 10,0% en poids d'une substance véhicule lipidique et de 0,05% à 5,0% en poids d'un tensioactif cationique.

**12.** Composition pour le soin des cheveux selon la revendication 11, dans laquelle le tensioactif cationique est un sel d'ammonium quaternaire.

**13.** Composition pour le soin des cheveux selon la revendication 12, dans laquelle la substance véhicule lipidique est choisie parmi l'alcool cétylique, l'alcool stéarylique, le palmitate de cétyle, le mnostéarate de glycéryle et leurs mélanges.